# EUROPEAN PATENT APPLICATION

(11) **EP 4 806 352 A1**
(43) Date of publication of application: **16.09.2026**
(21) Application number: 26164098.1
(22) Date of filing: 11.03.2026
(51) Int. Cl.: A61B 5/1455

(54) **NON-INTRUSIVE FAULT DETECTION FOR SENSOR CABLES OF PHYSIOLOGICAL MONITORS**

(30) Priority: 13.03.2025 US 202563771450 P
(71) Applicant: Draeger Medical Systems, Inc., Andover, MA 01810 (US)
(72) Inventor: Aiyawar, Vijaya K., Chester, 03036 (US)
(74) Representative: Haseltine Lake Kempner LLP

(57) **Abstract**

A compact, economical circuit assembly may detect and diagnose a variety of sensor cable faults in medical monitoring systems. The fault detection may continuously provide real-time information while consuming only minimal extra power. A dedicated local processor in the circuit assembly may communicate with a main processor of the medical monitor for more detailed analysis, long-term logging, and optional adaptive prediction, of short circuits, open circuits, and ground faults. The fault detector is suitable for adding on to existing medical monitors as well as building into new ones. A programmable portion of the circuit may be updated over a network to support new test algorithms and new types of cables and sensors.

## Description

### TECHNICAL FIELD

The present disclosure relates generally to the field of physiological monitoring. More particularly, it relates to detection of cable faults in the sensor cables of physiological monitoring systems.

### BACKGROUND

As context to aid in understanding the disclosure, this section includes information about potentially related art. This art may or may not qualify as "prior art" under the patent law of any given jurisdiction. Therefore, this section should not be construed as an "admission of prior art" under any relevant law.

Cables in physiological monitoring systems are routinely subjected to many forms of mechanical stress: tensile when they are pulled, compressive and/or shearing when they are pinched, bending when they are wound and unwound, *etc.* Sterilization, where applicable, may additionally expose them to temperature and humidity cycles, chemicals, and radiation. Their terminals may be connected and disconnected hundreds, even thousands, of times.

Clinicians and their staff may receive training in how to handle cables, and may ordinarily exercise care. However, when a patient's needs are urgent, equipment can become an obstacle course for hurrying caregivers. In these circumstances, lightweight flexible cables and their connections may become especially vulnerable.

If a monitor does not seem to be collecting data immediately after its sensor is coupled to a patient, swapping out a cable may therefore be the first step in a troubleshooting procedure. This requires locating a replacement cable and being able to trust that it functions correctly. When seconds may be of the essence in effectively treating the patient, the process may be more of a burden than would ordinarily be expected.

If all the cables function when first connected, they may still fail later. Depending on the type of sensor, the patient's condition, and the capabilities of the monitoring system, an undesirably long time may elapse before a human caregiver notices the failure and replaces the cable.

To underscore the undesirability of cable failures, regulatory bodies such as the International Standards Organization ("ISO") have begun to implement rules requiring built-in cable fault detection for certain types of sensors. For example, pulse oximeter (SpO₂) sensors measure blood oxygenation by non-invasively illuminating arteries with at least two different light-emitting diode ("LED") wavelengths and measuring the light transmitted through the arteries with at least one photodetector ("PD"). ISO 80601-261:2019 requires SpO₂ probes to include an electrical circuit to detect and announce faults related to short circuits ("shorts") and open circuits ("opens") connected to the LED(s) and PD(s).

One historical approach has been for health-care facilities to assign technicians to periodically test cables in isolation, with standard tools such as multimeters. This can be a time-consuming process. It also presupposes that enough spares are available that some cables could always be rotated out of use for testing long before they might be expected to fail. It also may not detect some "near-failure" conditions, in which the cable may still pass a test under relatively gentle lab conditions but may be weakened enough to fail during the next demanding real-life use. Additionally, the test circuit is not a circuit that detects and announces faults while the sensor continues to measure.

Some sophisticated analog front-ends ("AFEs") have been developed that offer an all-in-one solution for particular measurement types, such as the Texas Instruments^{®} AFE 4403 for optical heart-rate monitors and low-cost pulse oximeters. Its single chip includes a diagnostic circuit for detecting LED- and PD-related opens and shorts as well as a dual LED driver and a PD receiver. Its relatively high cost can be factored into a new device designed around it *ab initio,* but may be less suitable for retrofitting fault detection onto an existing sensor and monitor.

A fault detection module could alternatively be built from discrete off-the-shelf components such as (1) a controller with a state machine and a communications ("comms") interface; (2) a switch matrix; (3) a load resistance network; (4) an analog-to-digital converter ("ADC"); and a comparator. Though the cost would be lower than an all-in-one AFE and there would be flexibility in choosing the various components to work well with existing sensor and monitor electronics, such solutions may consume more board space and power than might be desired,

Therefore, the field of physiological monitoring would benefit from compact, low-power, low-cost detection of cable faults in the sensor cables of physiological monitoring systems that could be added onto an existing monitor to increase reliability, comply with emerging regulations, or both.

### SUMMARY

The following simplified summary is intended to provide a basic understanding of some aspects of the disclosure that may be included in the appended claims. It is not meant as an exhaustive overview of the subject matter, a complete list of key or critical elements, or a recitation of the limits of what may be claimed. Its only purpose is to introduce some of the concepts and terminology to be discussed in more depth in the Detailed Description.

The solutions disclosed herein may detect cable faults in various operating modes, including while the connected sensor actively measures one or more physiological parameters on a patient.

A physiological monitor may include a programmable digital logic block. The programmable digital logic block may include a state machine programmed to select a fault test and to transmit a corresponding test signal. The physiological monitor may also include a programmable analog logic block. The programmable analog logic block may include an analog switch matrix programmed to (1) receive a test signal from the state machine (2) apply the test signal to a cable coupling a sensor to the physiological monitor, and (3) receive a response.

The physiological monitor may also include (1) a programmable load programmed to receive the test signal and the response and transmit a corresponding voltage, (2) a comparator on the programmable digital logic block to receive the voltage from the programmable load, to convert the voltage to a test result, and to transmit the test result to the state machine, and (3) a communication interface on the programmable digital logic block to transmit the test result from the programmable digital logic block to at least one of a monitor processor and a monitor memory.

Some embodiments of the monitor may also include a local memory block. The programmable analog logic block, the programmable digital logic block, and the local memory block may occupy a footprint of between 2 and 20 square millimeters ("mm²"). The power requirements of some embodiments of the monitor, for all states of operation, may be supplied by a 1-6V power source. The programmable digital logic block may consume less than 3 *µA* in a sleep mode while the programmable analog logic block remains active. The communication interface may exchange data with the monitor processor or the monitor memory through a two-wire serial communication bus.

Some embodiments of the monitor may be operated by a method that may include coupling a cable to a sensor and a physiological monitor, positioning the sensor to measure a physiological parameter of a patient, measuring the physiological parameter while activating a first circuit in a programmable switch matrix, performing a first cable test using the first circuit, receiving a first test result from the cable, generating a first fault flag corresponding to the first test result, and transmitting the first test result to at least one of a fault register, a state machine, or a monitor processor.

In some embodiments of the method, at least part of the sensor's measurements of the physiological parameter may continue uninterrupted during at least part of the first cable test. The first circuit may be activated by being created or modified in a macro model. The macro model may be updated or modified the macro *in situ* at an end-user facility. At least one of the fault register and the state machine may be part of a programmable digital logic block, which may be updated or modified *in situ* at an end-user facility. The operation of the physiological monitor may begin with a start-up sequence and end with a shut-down sequence. The operation may also include loading the macro model from a non-volatile memory during the start-up sequence and saving the macro model to a non-volatile memory during the shut-down sequence.

The method may also include receiving instructions to perform a second cable test. The second cable test may differ from the first cable test and include activation of a second circuit, different from the first circuit, on the programmable analog logic block. The first cable test and the second cable test may be part of a plurality of cable tests, and each cable test may have a corresponding circuit to activate on the programmable analog logic block. Each of the plurality of cable tests may be repeated after either (1) passage of a predetermined time, (2) completion of a predetermined sequence of other tests or other processes by the monitor, or (3) selection of the cable test by the monitor processor according to an algorithm or adaptive learning model. The plurality of cable tests may include at least two of a short-circuit test, an open-circuit test, and a ground-fault test.

In some embodiments, the sensor may include a pair of back-to-back diodes, and the plurality of cable tests may include detecting a short circuit or an open circuit by measuring a direction of current in the cable to or from the back-to-back diodes.

A non-transitory computer-readable storage medium may contain instructions that, when executed, cause a physiological monitor processor to detect whether a sensor is coupled by a cable to a physiological monitor that includes the physiological monitor processor; decide to test the cable for faults if predetermined conditions are present; activate a cable fault detector; select a cable test from a group of tests stored in a memory; command the cable fault detector to perform the cable test; receive a pass-or-fail result of the cable test from the cable fault detector; and add the pass-or-fail result, an identifier of the cable test, and the date and time to a record of cable tests; and, if the pass-or-fail result is a fail, trigger an alert to a user.

In some embodiments of the non-transitory computer-readable storage medium, the cable test is selected based on the relative frequency of failure types stored in the record of cable tests. Some embodiments may also include receiving an update to the instructions for operating the cable fault detector, authenticating the update, and modifying the instructions according to the update. The modifying may include reprogramming a portion of a programmable analog logic block or a programmable digital logic block of the cable fault detector.

A physiological monitoring system may include a sensor to collect data representing a physiological parameter of a patient; a physiological monitor including a monitor processor to analyze the data; a cable coupled to convey the data from the sensor to the physiological monitor; and a cable fault detector to test the cable while the physiological monitoring system is operating. The cable fault detector may be located externally to the monitor processor, and may include a programmable analog logic block, a programmable digital logic block, and a group of components contained on one or both of the programmable analog logic block and the programmable digital logic block. The group of components including a state machine, an analog switch matrix, a load, and a comparator.

### BRIEF DESCRIPTION OF THE DRAWINGS

The drawings illustrate specific implementations described in detail by way of example. Neither the drawings nor the corresponding descriptions are intended to limit the scope of protected subject matter. On the contrary, the intent is to protect all modifications, equivalents, and alternatives falling within the scope of the appended claims.
FIG. 1A schematically illustrates a use case of a physiological monitoring system with an embedded cable fault detector and FIG. 1B schematically illustrates a portion of the cable fault detector of FIG. 1A in further detail according to some embodiments.
FIG. 2 is an architectural block diagram of a cable fault detector according to some embodiments.
FIGs. 3A-3E are detailed views of examples of some of the architectural blocks from FIG. 2.
FIGs. 4A-4H show examples of cable-test circuits created as needed in the switch matrix of FIGs. 2 and 3 according to some embodiments.
FIG. 5 is an example fault table or fault dictionary for the various fault tests.
FIG. 6 is a flowchart of an example method of detecting cable faults while monitoring a patient's physiological parameters according to some embodiments.

In the drawings, like reference numbers generally indicate elements that are functionally similar, structurally similar, or both.

### DETAILED DESCRIPTION

Unless otherwise defined, all terms including technical and/or scientific terms used herein have the same meaning as commonly understood by one of ordinary skill in the art of the present disclosure. Definitions of some art-specific terms as used in this document are presented below. Definitions of more general or art-agnostic patent terms are presented near the end of the document.

"Computer" as used herein may be any known computing apparatus or device, integrated or distributed, physical or virtual, digital or analog, using binary, ternary, decimal, hexadecimal, or any suitable notation. It may include a way to process data (a "processor"), a way to enter input, a way to access output, and a way to store or retrieve non-transitory instructions or data.

"Controller" as used herein may be a component or group of components that manages, directs, commands, or regulates the activities of one entity according to inputs from another entity, manipulating the flow of information between the two entities to automate a process according to one or more rules. The rules may be predetermined, or they may be adaptable as part of a machine-learning operation. The component may be realized as hardware, software, or a combination of both. The control may be closed-loop (collecting and feeding back previous results to inform subsequent refinements) or open-loop (not collecting results). "Microcontrollers" are a particular type of controller, see definition below,

"Link," in a programming context, may mean any association or relationship between two or more pieces of information that can be sensed and acted upon by a running program. The link may reside in a non-transient data structure or in a set of processing instructions. The link need not be a distinct item of code but may be a proximity or relative position of the two pieces of information, as in a table or register. By contrast, in the context of communications hardware, a "link" may be any suitable channel over which the communicating components may send or receive data.

"Logic blocks," which may be digital, analog, hybrid (including analog and digital portions), programmable (also called "configurable" in the art), or "hard" (pre-programmed and not rewriteable), may be physical units occupying a defined space, or may be functional units working together from distributed locations.

A "machine" herein may be any device, physical or virtual, that transmits or modifies any type of energy to perform or assist in the performance of human tasks.

A "macro model" may be broadly defined as a programmatic simulation of an electronic circuit or circuits. This disclosure is particularly concerned with macro models that can be input to an FPGA or other PLD (defined later in this section) to configure programmable logic hardware that will function as the described circuit. Some macro models represent common design blocks such as op-amps, memory, or even entire processors, and are designed to work with each other in a variety of configurations. Some macro models may be exported from one PLD and imported into another for reuse in a different design.

A "microcontroller" as used herein may be a controller integrated in a single chip, optionally with other components. Those typically discussed in the art have processing capability and memory divided into a program execution section and a register processing section. The program execution section contains program memory, instruction register(s), and control logic to store, decode, and execute the program. The register processing section contains the special function registers (SRFs) used to control the processor operations, including port registers used for input and output, and a program counter register to keep track of the program sequence by storing the address of the instruction currently being executed. Either section may include volatile memory, non-volatile memory, or both, but often the program execution section is largely non-volatile and the register processing section is largely volatile. Optionally, the microcontroller may include one or more timers, clocks, and ADCs.

A "module" may include hardware, software, or a combination of both assembled to implement a desired function. A degree of interchangeability between software and hardware is well known in the art and finds applicability in technological contexts such as those disclosed herein. Thus, a module may be implemented in, for example, electronic logic circuits comprised of electronic components such as transistors, resistors, inductors, capacitors, etc. Alternatively, a module may be implemented in software in the form of executable instructions stored by a processor-based resource. Alternatively, a module may be implemented in an integrated circuit such as an ASIC, an EPROM, or an EEPROM. Alternatively, a module may combine some or all of these technologies.

An "optical probe" is a sensor that emits light from a light source into or through a patient's body and uses a photodetector to measure resulting emergent light. The resulting emergent light may be light from the light source that is altered by its passage through bodily tissues and fluids (transmitted, reflected, refracted, scattered in any direction, or the like). Alternatively, the resulting emergent light may be emitted by bodily tissues or fluids (or by an additive thereto such as a fluorescent marker dye) as a result of stimulation by the light from the light source. An optical probe may include one or more light sources and one or more photodetectors.

A "PLD" (programmable logic device) herein may be any microelectronic device (analog, digital, or hybrid) with at least one internal structure modifiable after manufacture by programming. The modification may be rewriteable or non-rewriteable. Among others, field programmable gate arrays ("FPGAs"), programmable systems-on-chip ("PSoCs"), and both simpler and more complex configurable logic blocks ("CLBs") are included.

An umbrella term "sensor light source" ("SLS") will be used herein to include LEDs, organic LEDs ("OLEDs"), chip-scale lasers such as vertical-cavity surface-emitting lasers ("VCSELs"), and any other light source suitable for embedding in a medical sensor.

A "sensor" herein may be a transducer which converts a physical quantity to be measured to an electric signal, for example, a current signal or a voltage signal. The physical quantity may for example comprise electromagnetic radiation (e.g., photons of infrared or visible light), a magnetic field, an electric field, a pressure, a force, a temperature, a current, or a voltage, but is not limited thereto.

Illustrative examples of the subject matter claimed below are disclosed. In the interest of clarity, not all features of an actual implementation may be described for every example in this specification. It will be appreciated that in the development of any such actual implementation, numerous implementation-specific decisions may be made to achieve the developers' specific goals, such as compliance with system-related and business-related constraints, which will vary from one implementation to another. Moreover, it will be appreciated that such a development effort, even if complex and time-consuming, would be a routine undertaking for those of ordinary skill in the art having the benefit of this disclosure.

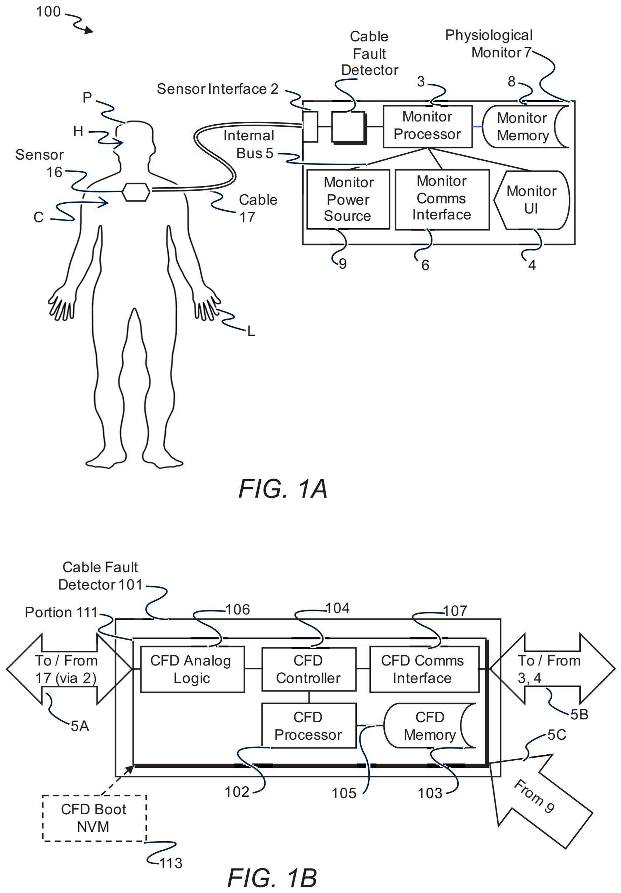

FIG. 1A schematically illustrates a use case of a physiological monitoring system with an embedded cable fault detector.

In use case 100 illustrated in FIG. 1A, sensor 16 is set up to collect at least one physiological parameter from patient P. In the illustrated example, sensor 16 is positioned on patient P's chest C. Additionally or alternatively, sensor 16 may be positioned on patient's head H, limb L, or other area of the body where the desired physiological parameter is measurable.

Sensor 16 may be an optical probe measuring SpO₂, photoplethysmography ("PPG"), blood glucose, or other light-responsive physiological parameters. Cable 17 may be a metal-clad or other shielded cable, a single-use cable, a sterilizable cable, a smart cable, or a hybrid cable connected to two or more sensors or carrying measurement data for two or more physiological parameters.

Physiological monitor 7 may receive data from sensor 16 through cable 17 at sensor interface 2, which may include one on more ports (not shown in this view) to which cable 17 may be detachably secured. The data signals received from sensor 16 may be analog signals. In such cases, sensor interface 2 may include a data acquisition circuit (not shown separately in this figure), which may include amplifying and filtering circuity as well as analog-to-digital conversion ("ADC") circuity that converts the analog signal to a digital signal for storage in monitor memory 8 and processing by monitor processor 3.

Physiological monitor 7 may include various modules interconnected by internal buses 5 to perform various functions. Each of the modules may include hardware, software, or a combination. Internal buses 5 may carry data signals, control signals, power, or combinations thereof between the various modules. In this description, internal modules of physiological monitor 7 include monitor processor 3, monitor user interface ("UI") 4, monitor communications ("comms") interface 6, monitor memory 8, and monitor power source 9. Other modules, though not illustrated here, may also be present.

Modules 3, 4, 6, 8, and 9 are described herein as "monitor" modules because they facilitate computing functions associated with the monitor. This does not preclude their controlling, being controlled by, or sharing resources with external devices connected through a network. Moreover, additional modules performing analogous functions may be located elsewhere, such as in a detachable monitor mount (not shown in this view), in a sensor 16, or in a smart cable 17. Such separate modules may additionally or alternatively be associated with specialized subsystems of the monitor, such as a power management subsystem or a cable fault detection subsystem.

A cable fault detector ("CFD") 101 may be positioned near (or, in some embodiments, may be wholly or partially integrated with) sensor interface 2. In some embodiments, cable fault detector 101 may include components or functions similar to the data acquisition circuit of sensor interface 2 or may process the incoming signals further in different ways. Cable fault detector 101 may perform one or more tests on cable 17 as described more fully below to detect any emergent cable faults. Such faults may include, by way of example and without limitation, short circuits, open circuits, or ground faults.

If cable fault detector 101 detects a fault, it communicates with monitor processor 3. Monitor processor 3, upon receiving notice of a cable fault, may alert a clinician or technician through a local device or a network. In some embodiments, monitor processor 3 may disable the sensor 16 associated with the malfunctioning cable 17 or send other control commands. Additionally, monitor processor 3 may log the incident in monitor memory 8, in a healthcare database, in a memory built into cable 17 in the case of smart cables, or elsewhere.

Cable fault detector 101 may repeat its cable fault tests one after the other in a set sequence, may be repeated at predetermined or adjustable intervals, or may be triggered by anomalies in a physiological parameter measurement. In some embodiments, a machine-learning or other adaptive model running on processor 3 or a different processor may learn an optimal timing or set of trigger events for repeating each of the tests.

One or more monitor processors 3 may organize and analyze physiological monitoring data received at sensor interface 2 and may also control the general operations of physiological monitor 7. Monitor processor(s) 3 may be any suitable processor-based resource. They may be, but are not limited to, a central processing unit ("CPU"), a hardware microprocessor, a multi-core processor, a single core processor, an FPGA, a controller, a microcontroller, an application specific integrated circuit ("ASIC"), a digital signal processor ("DSP"), a processor chipset including co-processors (either co-located or distributed among two or more chips, boards, or other locations), or other similar processing device capable of executing any type of instructions, algorithms, or software for controlling the operation and performing the functions of physiological monitor 7.

A monitor UI 4 may receive input from a user (for example, through a keyboard, a pointing device such as a mouse or trackpad, a touchscreen, a camera, a microphone, or a button) and provide output to a user (for example, through a display screen, an audio output, a haptic transducer, or an indicator light). The input may include commands, queries, interrupts, authentication, and ancillary data such as test identifiers. The output may include patient data, physiological parameters measured by the sensor, results of calculations, and hospital or patient care information.

A monitor comms interface 6 may permit physiological monitor 7 to communicate with one or more external devices or networks (not shown), either directly or indirectly. For example, monitor comms interface 6 may allow information output through monitor UI 4 to be output to a remote monitor (not shown). Additionally or alternatively, monitor comms interface 6 may send alert messages or other signals from alert system 10 to devices or destinations attended by clinicians. The communication may travel over wired or wireless links using any suitable protocol, including but not limited to Bluetooth^{®}, a cellular data, Wi-Fi^{®}, IEEE 802.11 protocol, Radio Frequency For Consumer Electronics ("RF4CE") protocol, and/or IEEE 802.15.4 protocol (e.g., Zigbee^{®} protocol).

Besides these different network connections, monitor comms interface 6 may permit direct (i.e., device-to-device) communications (e.g., messaging, signal exchange, etc.) between physiological monitor 7 and other devices such as a tablet, computer, or similar electronic device; or to an external storage device or memory. Such direct connections may use, for example, a universal serial bus ("USB") connection or other communication protocol interface.

Monitor memory 8 may receive data from monitor processor(s) 3 or other sources, store it for a short or long term in one or more structures, and allow monitor processor(s) 3 or other destinations to access the data. Monitor memory 8 may be a single memory device or a group of memory devices that are either co-located or distributed among multiple locations. The locations may include, without limitation, a chip shared with monitor processor 3 or any of the other functional modules of physiological monitor 7, or one or more separate memory modules. Monitor memory 8 may be removable, built-in, or a combination thereof, and may be volatile or non-volatile or a combination thereof. Monitor memory 8 may be, for example, a static or dynamic RAM, a memory buffer, a hard drive, a database, an EPROM, an EEPROM, a ROM, a flash memory, a hard disk, or any other computer readable medium capable of storing data and instructions. At least a portion of monitor memory 8 may be static RAM or a similar non-transitory storage medium that retains written content even when powered off.

Monitor power source 9 supplies power to at least some of the power-consuming components inside or connected to physiological monitor 7. Monitor power source 9 may include a self-contained power source such as a built-in rechargeable battery pack or a battery that can be detached and replaced. Additionally or alternatively, monitor power source 9 may include an interface to be powered through an electrical outlet or a wireless charger, either directly or through a monitor mount or other power connection system. A back-up power source (not shown separately) such as an uninterruptable power supply, a back-up battery, or a supercapacitor may be built in or connectable to the interface to provide continuous power to physiological monitor 7 during power failure, battery replacement, or other power loss.

Physiological monitor 7 may optionally be deployed as part of a larger health-facility management system with functions that may include, but are not necessarily limited to, physiological monitoring. The health-facility management system may include a group of physical virtual server and client devices connected in a network or cloud. Physiological monitor 7 may exchange information with health-facility management system over any suitable wired or wireless communication link.

FIG. 1B schematically illustrates a portion 111 of the cable fault detector 101 in further detail, according to some embodiments. Portion 111 may have several on-board functional modules communicatively coupled by CFD interconnects 105. CFD interconnects 105 may include buses, traces, vias, field couplings, or other conductive paths. Additionally or alternatively, they may be programmable interconnections enabled in a programmable logic device ("PLD") such as field-programmable gate array ("FPGA").

In those embodiments where portion 111 includes an FPGA or other PLD, some or all of the functional modules of portion 111 may be implemented as programmable analog logic blocks, programmable digital logic blocks, or programmable hybrid logic blocks. Each module may be contained in a single logic block, distributed over more than one logic block, or-co-located with other modules or parts of modules in a logic block.

The modules may include CFD processor 102, CFD memory 103, CFD controller 104, CFD comms interface 107, and CFD analog logic 106. In some embodiments, CFD processor 102 and CFD memory 103 may be implemented in partial, single, or multiple programmable digital logic blocks; CFD analog logic 106 may be implemented in partial, single, or multiple programmable analog logic block or blocks; CFD controller 104 and CFD comms interface 107 may be implemented in either digital or hybrid programmable logic blocks, depending on the types of signals they send and receive.

In some embodiments, all modules 102-107 may be contained in a PSoC that includes blocks of analog logic and digital logic (for purposes herein, a hybrid logic block may be equivalently described as a combination of an analog logic block and a digital logic block). In other embodiments, one or more of modules 102-107 may be communicatively coupled from another position in the monitor electronics system (for example, CFD analog logic 106 may be on a programmable analog logic block of a neighboring FPGA or simpler PLD, or CFD memory 103 may be in a programmable digital logic block mounted on top of the chip, over a bridge, in a package-on-package ("PoP") arrangement. In addition, some of the programmable logic blocks making up modules 102-107 may be so-called "hard blocks" fabricated into the PSoC by the PSoC manufacturer, rather than being programmed into the logic blocks. Hard blocks trade off some of the wide flexibility of programmable blocks for the (as of this writing) better performance and smaller footprints of ASICs. Processors, memory, controllers, and transceivers are non-limiting examples of hard-block modules included in some PSoCs because of their applicability to a wide range of devices. Among the CFD modules, CFD processor 102 executes the instructions for testing cable 17 to determine the presence or absence of various cable faults. The instructions may be stored in CFD memory 103 or communicated from monitor memory 8 through monitor processor 3, monitor processor bus 5B, and CFD comms interface 107. In some embodiments, a user interacting with monitor user interface 4 (or a remote network UI) may trigger instruction execution or load in new instructions. CFD processor 102 may direct CFD controller 104 to send detected faults and other test results through monitor processor 3 through CFD comms interface 107 and monitor processor bus 5B. In some embodiments, monitor processor bus 5B may be a two-wire serial bus such as an I²C, UART, SBI bus, or any suitable successor.

CFD processor 102 may be any suitable processor-based resource. It may be, but is not limited to, a hardware microprocessor, a multi-core processor, a single core processor, an FPGA, a controller, a microcontroller, an application specific integrated circuit ("ASIC"), a digital signal processor ("DSP"), a processor chipset including co-processors (either co-located or distributed among two or more chips, boards, or other locations), or other similar processing device capable of executing any type of instructions, algorithms, or software for controlling the operation and performing the functions of cable fault detector 101.

When CFD processor 102 executes a cable test, CFD controller 104 causes CFD analog logic 106 to apply signals to, or collect signals from, cable 17 through Interface/cable bus 5A and sensor interface 2. Meanwhile, power may be supplied from monitor power source 9 through monitor power bus 5C (which may, in some embodiments, be the same as monitor processor bus 5B). Alternatively, cable fault detector 101 may be powered from some other source. Optionally, programmable-logic embodiments of cable fault detector 101 may store its structural programming on an offboard non-volatile memory ("NVM") such as CFD boot NVM 113. The PSoC, FPGA, or other PLD may return to its factory state (including hard when powered down and retrieve its custom configuration from CFD boot NVM 113 when powered back on. CFD boot NVM 113 may also be configured to receive updates and other changes to the CFD structural programming through monitor user interface 4 or through a connected network.

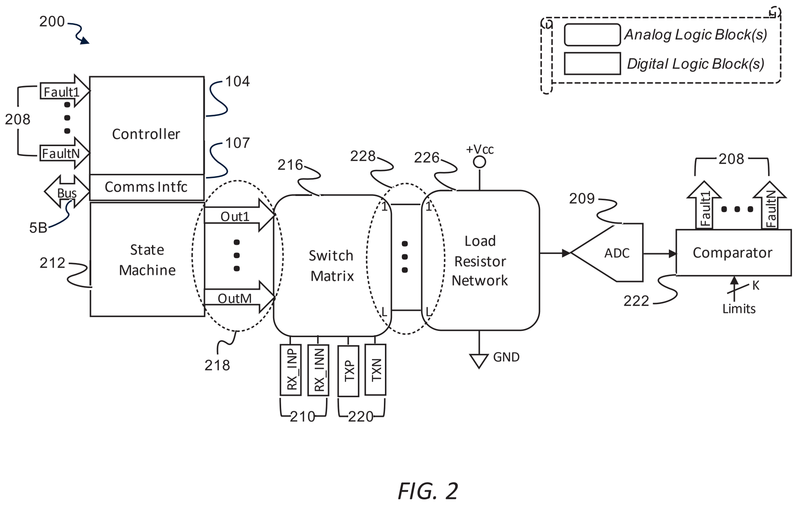

FIG. 2 is an architectural block diagram 200 of a cable fault detector according to some embodiments. As noted in the key section near the top right, in the illustrated embodiments the components drawn as rectangles with sharp corners (controller 104, comms interface 107, state machine 212 and comparator 208) may be implemented as partial, single, or multiple programmable digital logic blocks, while the components drawn as rectangles with rounded corners (switch matrix 216 and load resistor 226) may be implemented as partial, single, or multiple programmable analog logic blocks ADC 209 may be implemented as a programmable hybrid logic block with both analog and digital features. Any or all of the components may be newly designed macro models, or they may be existing macro models re-used from other devices in a new arrangement customized for medical sensor cable fault detection.

The use of programmable logic blocks permits the cable fault detector to occupy a small footprint, for example 2-20 mm². If a board in an existing physiological monitor has this amount of space available, a cable fault detector may be added as an upgrade to subsequent manufacturing runs of the board. New physiological monitors may be shipped with the upgraded board without necessarily changing other parts. Upgraded boards may be field-swappable into existing monitors. Alternatively, if existing boards have suitable empty sockets, a stand-alone cable fault detector may be field-installable in existing monitors as well as factory-installable in new monitors. Some embodiments may be at least partially reprogrammable in the field, such as *in situ* at an end-user facility. Existing macro models or other design blocks may be overwritten with new ones, or the relationships between the design blocks may be modified for new functions once the programmable logic block is added to the board. This can be particularly convenient if regulatory requirements change; a macro model upgrade could be performed quickly or even remotely, without taking medical monitoring equipment offline or out of the end-user facility for a significant length of time.

During a cable fault test cycle, CFD controller 104, which may be a digital logic block, receives the most recent fault flag(s) 208 from comparator 222. Each fault flag 208 may correspond to one of N predetermined faults. If no cable faults were found, all the flags may be null, or there may be a single "no faults" flag, or any other suitable no-faults behavior may be executed. CFD controller 104 may activate CFD comms interface 107 to send fault flags 208 and any optional accompanying information to monitor comms interface 6 (not shown in this view) over monitor processor bus 5B.

State machine 212, a digital logic block associated with CFD processor 102 (shown in FIG. 1), may transmit one or more of M programmed state machine outputs 218. They may include commands to take measurements associated with one or more of the N possible faults. M may differ from N because a test for a single fault may involve addressing more than one cable terminal, and other actions, such as waiting for instructions from monitor processor 3 (see FIG. 1A), may also be available. Input instructions may already reside on the state machine as a pre-programmed sequence, or may be received through CFD comms interface 107 over monitor processor bus 5B from the monitor processor 3, monitor UI 4, or a networked remote device accessed through monitor communication interface 6 (see FIGs. 1A and 1B).

Switch matrix 216 may be an analog logic block, a digital logic block containing one or more digital-to-analog converters ("DACs," not shown in this view), or a hybrid logic block including analog and digital portions. Switch matrix 216 may receive state machine outputs 218 from state machine 212 and differential analog inputs 210 from the cable terminals. Responding to these inputs, switch matrix 216 manipulates its internal switches to activate one or more of its L selectable pins 228 (L optionally being a number unequal to M or N), transmit an analog output signal on differential outputs 220, or both.

Load resistor network 226, which may be an analog logic block, may function as a programmable load by reading the states of selectable pins 228, applying a corresponding predetermined load resistance, and delivering the analog result to analog-to-digital converter ("ADC") 209 for conversion to a digital signal and input to digital comparator 222. K channels of limits (K optionally being a number unequal to L, M or N) are also input to digital comparator 222. From these inputs, digital comparator 222 transmits fault flags 208 as an output, which is transmitted to CFD controller 104 to begin the next cycle.

Any of CFD controller 104, CFD comms interface 107, state machine 212, switch matrix 216, load resistor network 226, ADC 209, or digital comparator 222 may be pre-programmed, field-programmable, or a partial combination of both. All of CFD controller 104, CFD comms interface 107, state machine 212, switch matrix 216, load resistor network 226, ADC 209, and digital comparator 222 may reside on a single chip which may be an FPGA, a PSoC, or another suitable type of PLD. Alternatively, some may reside on separate chips coupled by buses, bridges, or other interconnects.

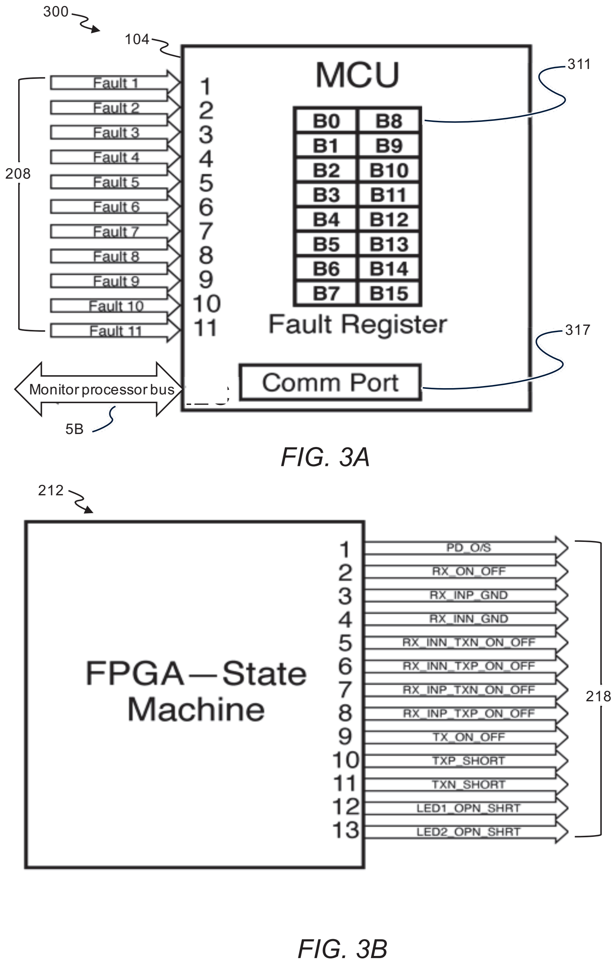

FIGs. 3A-3E are detailed views of examples of some of the architectural blocks from FIG. 2. In FIG. 3A, block 300 combines CFD controller 104 (a microcontroller "MCU") and a communications port ("Comm Port") 317 in a digital logic block. Either or both may be field-programmable or hard-coded. Fault flags 208 may enter CFD controller 104 (here, the number N of predetermined fault states is 11, but other numbers N may be chosen depending on the number of possible faults in the attached cable). A value representing a description of the fault (its type and, in some embodiments, its location) may be written to fault register 311, a dedicated hardware register inside CFD controller 104. Information about the fault may also be sent out of the cable fault detector through comm port 317 and monitor processor bus 5B to (the following are not shown in this view) monitor processor 3, monitor user interface 4, or a remote device reachable through monitor comms interface 6.

In FIG. 3B, state machine 212 is a digital logic block that constitutes part of the CFD processor (102 in FIG. 1B) functionality. State machine 212 may use both past and present information from fault register 311 (shown in FIG. 3A) as well as other information to calculate state machine outputs 218 (see FIG. 3B). Here, the number M of different state machine outputs 218 is 13, but other numbers of state machine outputs 218 may be chosen.

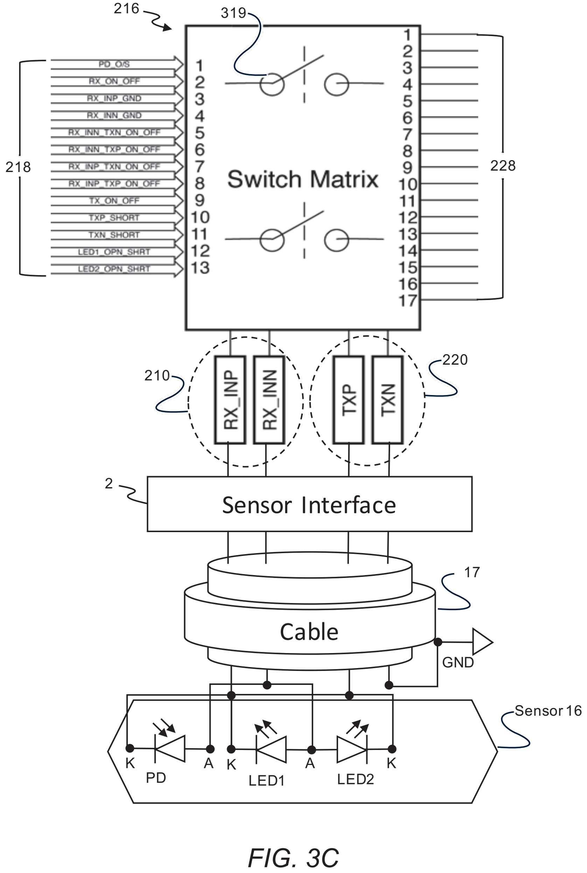

FIG. 3C shows an example of the cable fault detector's switch matrix 216. Its connections through the monitor's sensor interface 2 (if present as a separate component) to cable 17, and cable 17's connections to an example sensor 16. Switch matrix 216 may be an analog logic block or a digital logic block, and may be part of a PSoC or packaged separately. State machine outputs 218 cause switch matrix 216 to configure its multiple switches 319 into a corresponding test circuit (not shown here, but some examples will be shown in other FIGs).

The test circuit applies signals to sensor cable 17 through differential outputs 220 ("TXP," "TXN"), either directly or through sensor interface 2. Responsive signals return from cable 17 to differential inputs 210 ("RX_INP," "RX_INN"). The sensor 16 illustrated here is an optical probe with a diode-type photodetector (PD) and a dual-LED package where LED1 and LED2 share an anode A but have separate cathodes K. Cable 17 is shown with an internal ground connection. If the return signal through differential inputs 210 indicates a fault (short, open, or unwanted ground), switch matrix 216 provides signals to its selectable pins 228 to configure a programmable load in load resistor network 226. As illustrated, the number L of selectable pins 228 is 17, but other numbers of selectable pins 228 may be chosen.

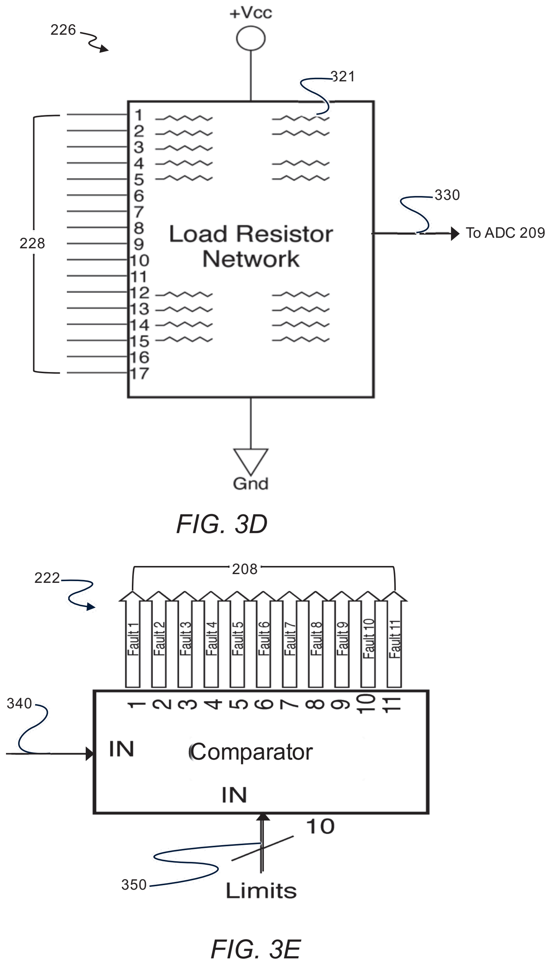

FIG. 3D shows load resistor network 226, an analog logic block. Load resistor network 226 may include multiple resistors 321 in a single package (surface mount, through-hole, or other suitable type). Resistors 321 may have different values or the same value, and may be isolated from each other or connected in an R2R ladder (digital to analog converter), bussed, voltage divider, dual terminator, digital-to-analog converter, or decade resistor circuit type. In some embodiments, the connections between resistors 321 may be selectable, reconfigurable, or field-programmable. The load resistance determining analog output 330 to be sent to ADC 209 may be determined by the configuration of the selectable pins 228 connected from switch matrix 216.

Load resistor network 226, as illustrated, may be connected to ground ("Gnd") and to a voltage source ("V_{cc}"), a standard positive voltage source for bipolar junction transistors. Other embodiments may use other suitable voltage or current sources.

FIG. 3E is an example of digital comparator 222 according to some embodiments. Analog output 330 may be digitized by ADC 209 (not shown in this view) and the resulting digital signal becomes digital input 340 for digital comparator 222. Predetermined or calculated comparator limits 350 are provided through an interconnect. Here, the number of channels K in the limits interconnect is 10, but other suitable numbers may be used. From digital input 340 and comparator limits 350, digital comparator 222 generates the fault flags 208 for routing back to the MCU to complete a fault-testing cycle.

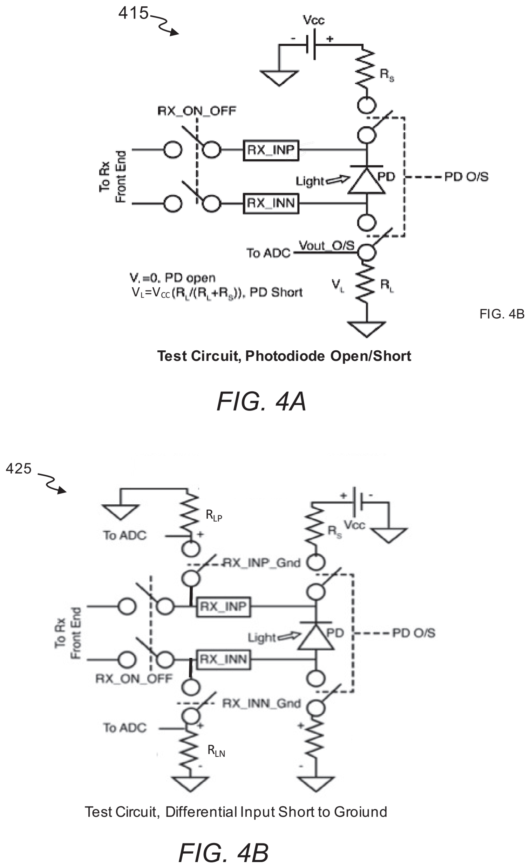

FIGs. 4A-4H show examples of cable-test circuits created as needed in the switch matrix of FIGs. 2 and 3 according to some embodiments. In these examples, the cable connects a monitor to an optical probe sensor with two LEDs and a PD. The sensor may be, for example, an SpO₂ non-invasive blood oxygenation sensor with two LEDs of different wavelengths, such as one visible red and one infrared, and a photodetector that can measure either of the wavelengths during operation. Note that these figures are electrical schematics that may or may not correspond to physical circuit layouts.

FIG. 4A is a circuit diagram of an example of a test circuit 415 to detect opens and shorts in the photodiode or its connections. The four switches may be part of the switch matrix of the cable fault detector. During normal operation, the operating (RX_ON_OFF) switches may be closed, and the testing (PD O/S) switches may be open. To isolate the photodiode circuit, including RX_INP, PD, and RX_INN, the testing (PD O/S) switches may be closed, and the operating (RX_ON_OFF) switches may be opened. The test may include reading voltage V_{L} across load resistor R_{L}. If the photodiode path includes an open circuit, V_{L}=0 because no current from the test-circuit voltage source reaches *R_{L}.* If the photodiode is shorted, ${V}_{L} = {V}_{CC} \left(\frac{{R}_{L}}{\left({R}_{L}+{R}_{S}\right)}\right)$, where *V_{CC}* is the voltage supplied by the test-circuit voltage source and *R_{S}* is a known test-circuit resistance in series with the test-circuit voltage source and upstream of photodiode PD. In other words, if PD is shorted, the test circuit behaves like a voltage divider with *V_{CC},R_{L},* and *R_{S}* in series. If neither fault is present, *V_{L}* is neither 0 nor ${V}_{CC} \left(\frac{{R}_{L}}{\left({R}_{L}+{R}_{S}\right)}\right)$. If more than one photodiode is connected to the cable under test, each photodiode will have its own independent test circuit.

FIG. 4B is a circuit diagram of an example of a test circuit 425 to detect shorts to ground (aka ground faults) in differential inputs RX_INP and RX_INN or their connections. The six switches may be part of the switch matrix of the cable fault detector. During normal operation, the operating (RX_ON_OFF) switches may be closed and the testing (PD O/S x2, RX_INP_GND, and RX_INN_GND) switches may be open. Testing the RX_INP differential input may include opening the RX_ON_OFF switches and closing the RX_INP_GND and top PD O/S switches to isolate a branch including RX_INP.

If RX_INP is shorted to ground, no current will flow in the isolated branch and no voltage change will be measured across load resistor R_{L,P}. Similarly, testing the RX_INN differential input may include opening the RX_ON_OFF switches and closing the RX_INN_GND and bottom PD O/S switches to isolate a branch including RX_INN. If RX_INN is shorted to ground, no current will flow in the isolated branch and no voltage change will be measured across load resistor R_{L,N}. If neither fault is present, R_{L,P} and R_{LN} will show an expected current flow and voltage change when connected in isolation to a voltage source and to ground.

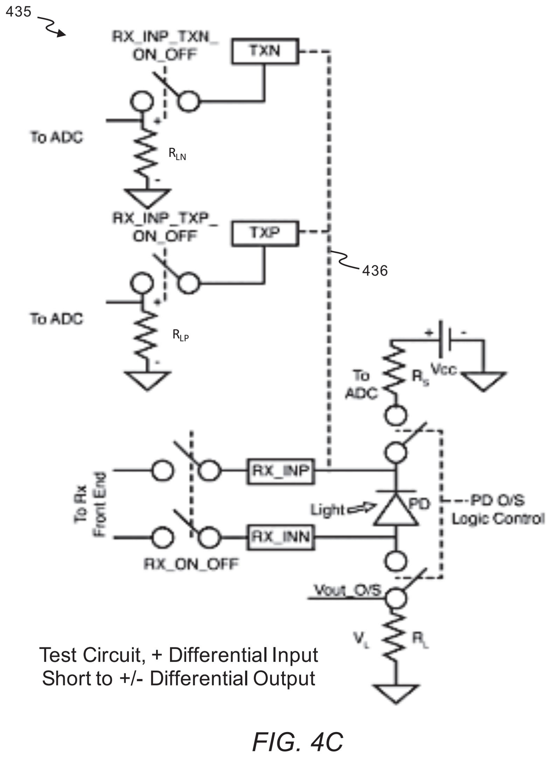

FIG. 4C is a circuit diagram of an example of a test circuit 435 to detect shorts between positive differential input RX_INP and at least one of differential outputs TXP or TXN. The six switches may be part of the switch matrix of the cable fault detector. During normal operation, the operating (RX_ON_OFF) switches may be closed and all the other switches shown in this figure may be open. To run the test, the switches are configured to temporarily connect voltage source *V_{CC}* to RX_INP while isolating RX_INP from the rest of the circuit. If RX_INP is shorted to TXP, TXN, or both, applying *V_{CC}* across RX_INP will cause current to flow in unwanted leakage path(s) 436 when switch RX_INP_TXP_ON_OFF or switch RX_INP_TXP_ON_OFF is closed, causing a non-zero voltage across R_{L,P} and/or R_{LN}. If no faults are present, the voltage across R_{L,P} and R_{LN} will be zero despite applying *V_{CC}* across RX_INP and closing RX_INP_TXP_ON_OFF or RX_INP_TXP_ON_OFF.

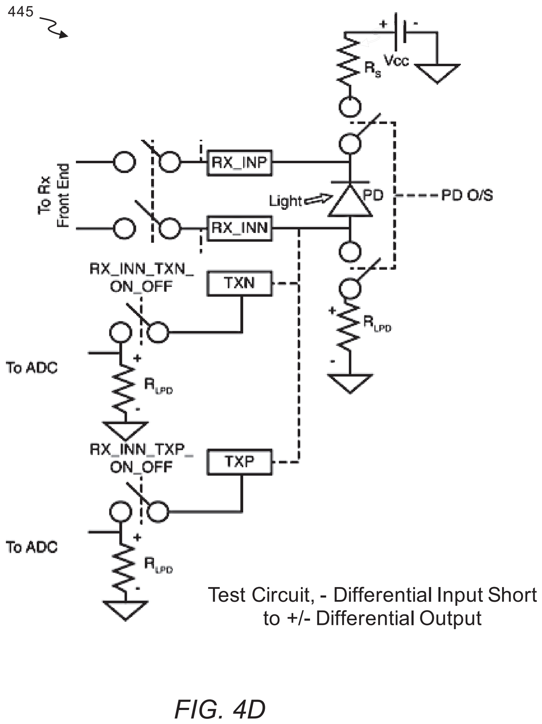

FIG. 4D is a circuit diagram of an example of a test circuit 445 to detect shorts between differential input RX_INN and a differential output, either TXP or TXN. The six switches may be part of the switch matrix of the cable fault detector. During normal operation, the operating (RX_ON_OFF) switches may be closed and all the other switches shown in this figure may be open. To run the test, the switches are configured to temporarily connect voltage source *V_{CC}* to RX_INN while isolating RX_INN from the rest of the circuit. If RX_INN is shorted to TXP, TXN, or both, applying *V_{CC}* across RX_INN will cause current to flow in unwanted leakage path(s) 446 when switch RX_INN_TXP_ON_OFF or switch RX_INN_TXP_ON_OFF is closed, causing a non-zero voltage across R_{L,P} and/or R_{LN}. If no faults are present, the voltage across R_{L,P} and R_{LN} will be zero despite applying *V_{CC}* across RX_INN and closing RX_INN_TXP_ON_OFF or RX_INN_TXP_ON_OFF.

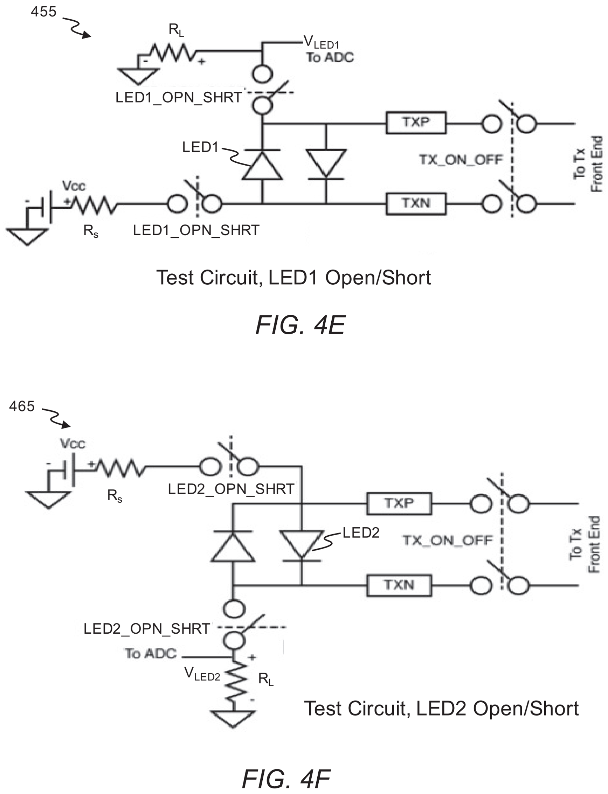

FIG. 4E is a circuit diagram of an example of a test circuit 455 to detect opens and shorts in the circuit of a first light source LED1. In this example, the light source may be an LED to emit a first wavelength, for example a visible or infrared LED. The four switches may be part of the switch matrix of the cable fault detector. During normal operation, the operating (TX_ON_OFF) switches may be closed and all the other switches shown in this figure may be open. To isolate the LED circuit, including TXP, LED1, and TXN, the testing switches (LED1_OPN_SHRT) may be closed, and the operating (TX_ON_OFF) switches may be opened.

The test may include reading voltage V_{LED1} across load resistor R_{L}. If the LED path includes an open circuit, V_{L}=0 because no current from the test-circuit voltage source reaches *R_{L}*. If the LED is shorted, ${V}_{L} = {V}_{CC} \left(\frac{{R}_{L}}{\left({R}_{L}+{R}_{S}\right)}\right)$, where *V_{CC}* is the voltage supplied by the test-circuit voltage source and *R_{S}* is a known test-circuit resistance in series with the test-circuit voltage source and upstream of LED1. In other words, if LED1 is shorted, the test circuit behaves like a voltage divider with *V_{CC}, R_{L},* and *R_{S}* in series. If neither fault is present, *V_{L}* is neither 0 nor ${V}_{CC} \left(\frac{{R}_{L}}{\left({R}_{L}+{R}_{S}\right)}\right)$. If more than one LED is connected to the cable under test, each LED may have its own independent test circuit.

FIG. 4E is a circuit diagram of an example of a test circuit 455 to detect opens and shorts in the circuit of a first light source LED1. In this example, the first light source may be an LED designed to emit a spectrum centered on a first wavelength, for example a visible or infrared wavelength. The four switches may be part of the switch matrix of the cable fault detector. During normal operation, the operating (TX_ON_OFF) switches may be closed and all the other switches shown in this figure may be open. To isolate the LED1 circuit, including TXP, LED1, and TXN, the testing (LED1_OPN_SHRT) switches may be closed, and the operating (TX_ON_OFF) switches may be opened.

The test may include reading voltage V_{LED1} across load resistor R_{L}. If the LED1 path includes an open circuit, V_{L}=0 because no current from the test-circuit voltage source reaches *R_{L}*. If LED1 is shorted, ${V}_{L} = {V}_{CC} \left(\frac{{R}_{L}}{\left({R}_{L}+{R}_{S}\right)}\right)$, where *V_{CC}* is the voltage supplied by the test-circuit voltage source and *R_{S}* is a known test-circuit resistance in series with the test-circuit voltage source and upstream of LED1. In other words, if LED1 is shorted, the test circuit behaves like a voltage divider with *V_{CC}, R_{L},* and *R_{S}* in series. If neither fault is present, *V_{L}* is neither 0 nor ${V}_{CC} \left(\frac{{R}_{L}}{\left({R}_{L}+{R}_{S}\right)}\right)$. If more than one LED is connected to the cable under test, each LED may have its own independent test circuit.

FIG. 4F is a circuit diagram of an example of a test circuit 465 to detect opens and shorts in the circuit of a second light source LED2. In this example, the second light source may be an LED designed to emit a spectrum centered on a second wavelength, for example a visible or infrared wavelength. As illustrated, LED2 and LED1 are wired back-to-back in a single package, but alternatively they may be in separate packages. The center wavelengths of LED1 and LED2 may be different from each other. The four switches may be part of the switch matrix of the cable fault detector. During normal operation, the operating (TX_ON_OFF) switches may be closed and all the other switches shown in this figure may be open. To isolate the LED2 circuit, including TXP, LED2, and TXN, the testing (LED2_OPN_SHRT) switches may be closed, and the operating (TX_ON_OFF) switches may be opened.

The test may include reading voltage V_{LED2} across load resistor R_{L}. If the LED2 path includes an open circuit, V_{L}=0 because no current from the test-circuit voltage source reaches *R_{L}*. If LED2 is shorted, ${V}_{L} = {V}_{CC} \left(\frac{{R}_{L}}{\left({R}_{L}+{R}_{S}\right)}\right)$*,* where *V_{CC}* is the voltage supplied by the test-circuit voltage source and *R_{S}* is a known test-circuit resistance in series with the test-circuit voltage source and upstream of LED2. In other words, if LED2 is shorted, the test circuit behaves like a voltage divider with *V_{CC}, R_{L},* and *R_{S}* in series. If neither fault is present, *V_{L}* is neither 0 nor ${V}_{CC} \left(\frac{{R}_{L}}{\left({R}_{L}+{R}_{S}\right)}\right)$*.* If more than one LED is connected to the cable under test, each LED may have its own independent test circuit.

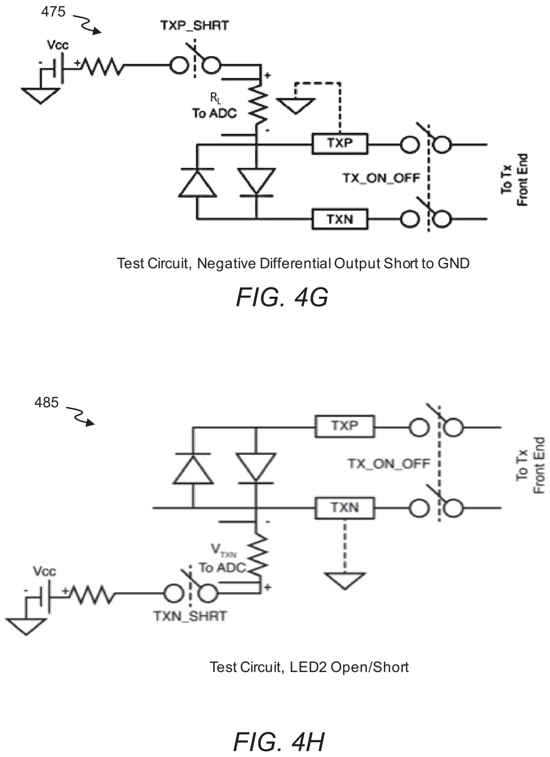

FIG. 4G is a circuit diagram of an example of a test circuit 475 to detect shorts to ground (aka ground faults) in positive differential output TXP or its connections. The three switches may be part of the switch matrix of the cable fault detector. During normal operation, the operating (TX_ON_OFF) switches may be closed, and the testing (TXP_SHRT) switches may be open. Testing the TXP differential input may include opening the TX_ON_OFF switches and closing the TXP_SHRT switches to isolate a branch including TXP and connect a known voltage source Vcc. If TXP is shorted to ground, no current will flow in the isolated branch and no voltage will be measured across load resistor R_{L}.

FIG. 4H is a circuit diagram of an example of a test circuit 485 to detect shorts to ground (aka ground faults) in negative differential output TXN or its connections. The three switches may be part of the switch matrix of the cable fault detector. During normal operation, the operating (TX_ON_OFF) switches may be closed, and the testing (TXN_SHRT) switches may be open. Similarly, testing the TX_INN differential input may include opening the TX_ON_OFF switches and closing the TXN_SHRT switches to isolate a branch including TXN. If TXN is shorted to ground, no current will flow in the isolated branch and no voltage change will be measured across load resistor R_{L,N}. If neither fault is present, R_{L,P} and R_{LN} will show an expected current flow and voltage change when connected in isolation to a voltage source and to ground.

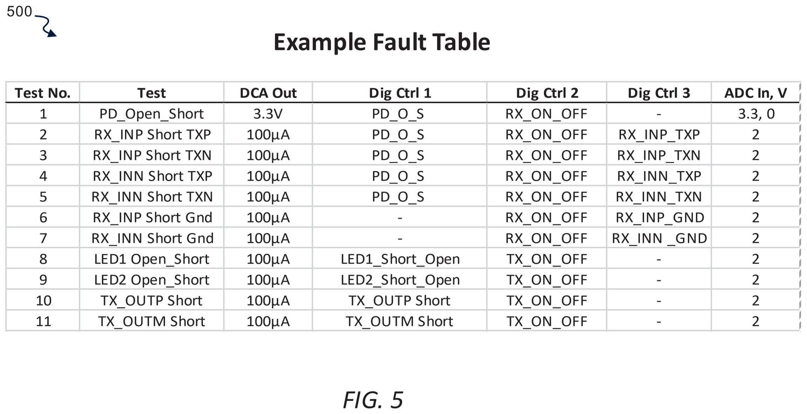

FIG. 5 is an example fault table or fault dictionary for the various fault tests. In fault table 500, tests 1-11 may use the test circuits described in FIGs. 4A-4H, respectively, or their equivalents, or other suitable circuits designed around the particular photodetector(s), light source(s), differential inputs, and differential outputs used in the sensor. The output column may include a voltage (3.3V) for the photodetector test 1 and a current (100µA) for the other tests. The digital columns Dig Ctrl 1, Dig Ctrl 2, and Dig Ctrl 3 may be used to identify the switches controlled in the switch matrix to perform each test. The "ADC In" column may list the voltage delivered to the analog-to-digital converter. The cable fault detector described in the table could be powered by a 4-6V source. To save power, the cable fault detector may be programmed to go into a sleep mode between test cycles, where the analog logic blocks continue to operate while the digital logic blocks stand by, consuming a reduced current such as 3 µA.

Different hardware embodiments may facilitate a variety of different methods for cable fault testing. Some typical operations may include coupling a cable to a sensor and to a physiological monitor; positioning the sensor to measure a physiological parameter of a patient; and measuring the physiological parameter. Meanwhile, the cable fault detector may execute at least one test cycle by activating a first circuit in a programmable switch matrix, performing a first cable test using the first circuit, receiving a first test result from the cable, generating a first fault flag corresponding to the first test result, and transmitting the first test result to one or more of a fault register, a state machine, or a monitor processor. In some embodiments, the physiological monitor may continue its parameter measurements uninterrupted while the cable fault detector executes one or more test cycles. In other embodiments, there may be one or more interruptions in data gathering, but such interruptions are much shorter than the expected variations in the physiological parameter being measured, such as 0.4-1.2 sec for a heartbeat or 0.1 second for a gamma brainwave.

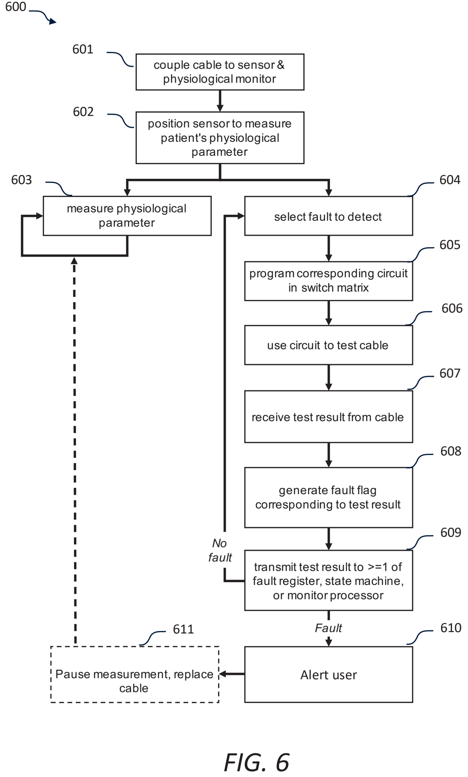

FIG. 6 is a flowchart of an example method of detecting cable faults while monitoring a patient's physiological parameters according to some embodiments. Use method 600 may begin with operation 601, coupling the sensor cable to a sensor and a physiological monitor in couple cable to a sensor and a physiological monitor. Operation 602 may involve positioning the sensor to capture a physiological parameter of a patient. For example, the sensor may be a fingertip SpO₂ probe, and the positioning may involve opening the clip, inserting the patient's finger, and allowing the clip to close. As another example, the sensor may be an ECG sensor with multiple leads and probes, and the positioning may involve placing each lead or probe on the patient's body according to a standardized arrangement, such as 6-lead or 12-lead ECG. From these examples, those skilled in the art will recognize how analogous variations on operation 602 may be implemented with other physiological sensor types. When all the probes for the sensor have been placed, operation 603 of measuring physiological parameters may commence. Measurement operation 603 is shown as a loop because the measurements may be continuous or periodically repeated.

While measurement operation 603 continues (without interruption in some embodiments), a particular fault test is selected in operation 604. The test may be selected by the local processor of a cable fault detector embedded in the physiological monitor (e.g., CFD processor 102 in FIG. 1B), by a main processor of the physiological monitor (e.g., monitor processor 3 in FIG. 1A), by a local or remote user such as a clinician, or any other suitable source. The selection may be a fixed sequence, cycling through all the tests in a predetermined order. Alternatively, the most likely faults may be checked more often than the less likely faults. As another alternative, a machine learning model on the monitor or at a networked location may repeatedly evaluate collected fault data and adapt a selection algorithm to fit any changed expectations inferred in the process. Each of the selectable tests may be associated with a corresponding test circuit in the programmable switch matrix and programmable load: Operation 605 may execute the program to set up the circuit. Operation 606 may use the programmed test circuit to test the connected sensor cable for the selected fault. Operation 607 may return the test result from the programmable circuit to an ADC. Operation 608 may feed the digitized test result to a comparator, which may generate a corresponding fault flag. Operation 609 may transmit the fault flag, and optionally other characteristics of the test result, to a fault register, a state machine, a monitor processor (e.g., monitor processor 3 in FIG. 1A), a remote processor with similar capabilities to monitor processor 3 in FIG. 1A, or any combination.

If no fault is detected, the system then returns to fault test selection operation 604, selects the same test or a different test, and repeats operations 605-609. If the test reveals a fault that may prevent the gathering of useful physiological data through the cable, an alert may be sent to a clinician in operation 610. Optionally, the measurement may be paused in operation 611 while the cable is replaced. In some embodiments, the gathering of data is not affected by the fault detection processes unless and until a fault is found. Some embodiments of the program may "remember" if a cable has just been replaced and, if the same fault is detected in the replacement cable, alert the user that the sensor itself may be causing the problem.

In some embodiments, additional operations may be added to the method, for example, a start-up sequence may be performed before the method, in which one or more macro models are loaded into the cable fault detector's programmable logic from a non-volatile memory such as CFD Boot NVM 113 in FIG. 1B. A shut-down sequence may be performed after the method, which may include saving one or more macro models to CFD Boot NVM 113. In embodiments where one or more of the macro models may have changed between start-up and shut-down, such as where the test selection may be modified by machine learning, at least one previous version of the macro model may be saved along with the latest version.

The illustrated order of these operations is intended as a non-limiting example. The order of some processes may be swapped, and/or some processes may proceed in parallel, without exceeding the scope of the claims.

In a first embodiment, a physiological monitor comprises a programmable digital logic block including a state machine programmed to select a fault test and to transmit a corresponding test signal; a programmable analog logic block including an analog switch matrix programmed to receive a test signal from the state machine, to apply the test signal to a cable coupling a sensor to the physiological monitor, and to receive a response, and a programmable load programmed to receive the test signal and the response and to transmit a corresponding voltage; a comparator on the programmable digital logic block to receive the voltage from the programmable load, to convert the voltage to a test result, and to transmit the test result to the state machine; and a communication interface on the programmable digital logic block to transmit the test result from the programmable digital logic block to at least one of a monitor processor and a monitor memory.

In a second embodiment, the monitor further comprises a local memory block, and the programmable analog logic block, the programmable digital logic block, and the local memory block occupy a footprint of between 2 and 20 square millimeters ("mm²").

In a third embodiment, the power requirements for all states of operation are supplied by a 1-6V power source.

In a fourth embodiment, the programmable digital logic block consumes less than 3 *µA* in a sleep mode while the programmable analog logic block remains active.

In a fifth embodiment, the communication interface exchanges data with the monitor processor or the monitor memory through a two-wire serial communication bus.

In a sixth embodiment, a method comprises coupling a cable to a sensor and a physiological monitor; positioning the sensor to measure a physiological parameter of a patient; and measuring the physiological parameter while activating a first circuit in a programmable switch matrix; performing a first cable test using the first circuit; receiving a first test result from the cable; generating a first fault flag corresponding to the first test result; transmitting the first test result to at least one of a fault register, a state machine, or a monitor processor.

In a seventh embodiment, at least part of the sensor's measurements of the physiological parameter continue uninterrupted during at least part of the first cable test.

In an eighth embodiment, the first circuit is activated by being created or modified in a macro model.

In a ninth embodiment, the method further comprises updating or modifying the macro model *in situ* at an end-user facility.

In a tenth embodiment, at least one of the fault register and the state machine is part of a programmable digital logic block, and further comprises updating or modifying the programmable digital logic block *in situ* at an end-user facility.

In an eleventh embodiment, the operating of the physiological monitor begins with a start-up sequence and ends with a shut-down sequence, and further comprises loading the macro model from a non-volatile memory during the start-up sequence, and saving the macro model to a non-volatile memory during the shut-down sequence.

In a twelfth embodiment, the method further comprises receiving instructions to perform a second cable test.

In a thirteenth embodiment, the second cable test differs from the first cable test and includes activation of a second circuit, different from the first circuit, on the programmable analog logic block.

In a fourteenth embodiment, the first cable test and the second cable test are part of a plurality of cable tests, each cable test having a corresponding circuit to activate on the programmable analog logic block.

In a fifteenth embodiment, the method further comprises repeating each of the plurality of cable tests is repeated after one of: passage of a predetermined time, completion of a predetermined sequence of other tests or other processes by the monitor; or selection of the cable test by the monitor processor according to an algorithm or adaptive learning model.

In a sixteenth embodiment, the plurality of cable tests comprises at least two of a short-circuit test, an open-circuit test, and a ground-fault test.

In a seventeenth embodiment, the sensor comprises a pair of back-to-back diodes and the plurality of cable tests comprises detecting a short circuit or an open circuit by measuring a direction of current in the cable to or from the back-to-back diodes.

In an eighteenth embodiment, a non-transitory computer-readable storage medium contains instructions that, when executed, cause a physiological monitor processor to: detect whether a sensor is coupled by a cable to a physiological monitor that includes the physiological monitor processor; decide to test the cable for faults if predetermined conditions are present; activate a cable fault detector; select a cable test from a group of tests stored in a memory; command the cable fault detector to perform the cable test; receive a pass-or-fail result of the cable test from the cable fault detector; and add the pass-or-fail result, an identifier of the cable test, and the date and time to a record of cable tests; if the pass-or-fail result is a fail, trigger an alert to a user.

In a nineteenth embodiment, selecting the cable test is based on the relative frequency of failure types stored in the record of cable tests.

In a twentieth embodiment, the non-transitory computer-readable storage medium of claim further comprises receiving an update to the instructions for operating the cable fault detector; authenticating the update; and modifying the instructions according to the update, wherein the modifying includes reprogramming a portion of a programmable analog logic block or a programmable digital logic block of the cable fault detector.

In a twenty-first embodiment, a physiological monitoring system comprises a sensor to collect data representing a physiological parameter of a patient; a physiological monitor including a monitor processor to analyze the data; a cable coupled to convey the data from the sensor to the physiological monitor; and a cable fault detector to test the cable while the physiological monitoring system is operating, the cable fault detector located externally to the monitor processor and including: a programmable analog logic block; a programmable digital logic block; and a group of components contained on one or both of the programmable analog logic block and the programmable digital logic block, the group of components including a state machine, an analog switch matrix, a load, and a comparator.

Unless otherwise defined, all terms including technical and/or scientific terms used herein have the same meaning as commonly understood by one of ordinary skill in the art to which the present disclosure pertains. In addition, unless otherwise defined, all terms defined in generally used dictionaries may not be overly interpreted. The following are specific definitions of terms used herein that are more common in usage than the specialized terms in the previous definition section.

As used herein, the article "a" or "an" is intended to have its ordinary meaning in the patent arts, namely "one or more."

The term "about" when applied to a value generally means within the tolerance range of the equipment used to transmit the value, or in some examples, means plus or minus 10%, or plus or minus 5%, or plus or minus 1%, unless otherwise expressly specified.

Use of the phrases "capable of," "capable to," "operable to," or "configured to" in one or more embodiments, refers to some apparatus, logic, hardware, and/or element designed in such a way to enable the use of the apparatus, logic, hardware, and/or element in a specified manner.

When an element is referred to as being "connected" or "coupled" to another element, it can be directly connected or coupled to the other element or intervening elements may be present. In contrast, when an element is referred to as being "directly connected" or "directly coupled" to another element, there are no intervening elements present. Other words used to describe the relationship between elements should be interpreted in a like fashion (e.g., "between" versus "directly between," "adjacent" versus "directly adjacent," etc.).

The terms "example," "examples," or "exemplary" indicate examples or instances and do not imply or require any preference for the noted examples. Thus, the present disclosure is not to be limited to the examples and designs described herein but is to be accorded the widest scope consistent with the principles and novel features disclosed.

Expressions such as "include" and "may include" which may be used in the present disclosure denote the presence of the disclosed functions, operations, and constituent elements, and do not limit the presence of one or more additional functions, operations, and constituent elements. In the present disclosure, terms such as "include" and/or "have", may be construed to denote a certain characteristic, number, operation, constituent element, component, or a combination thereof, but should not be construed to exclude the existence of or a possibility of the addition of one or more other characteristics, numbers, operations, constituent elements, components, or combinations.

The subject matter of the present disclosure is provided as examples of apparatus, systems, methods, circuits, and programs for performing the features described in the present disclosure. However, further features or variations are contemplated in addition to the features described above. It is contemplated that the implementation of the components and functions of the present disclosure can be done with any newly arising technology that may replace any of the above-implemented technologies.

The detailed description is made with reference to the accompanying drawings and is provided to assist in a comprehensive understanding of various example embodiments of the present disclosure. Changes may be made in the function and arrangement of elements discussed without departing from the scope of the disclosure. Various embodiments may omit, substitute, or add various procedures or components as appropriate. For instance, features described with respect to certain embodiments may be combined in other embodiments. In addition, descriptions of well-known functions and constructions may be omitted for clarity and conciseness. Accordingly, those of ordinary skill in the art will recognize that various changes and modifications of the examples described herein can be made without departing from the scope of the present disclosure.

Various modifications to the disclosure will therefore be readily apparent to those skilled in the art, and the generic principles defined herein may be applied to other variations without departing from the scope of the present disclosure.

## Claims

1. A physiological monitor, comprising:
a programmable digital logic block including a state machine programmed to select a fault test and to transmit a corresponding test signal;
a programmable analog logic block including:
an analog switch matrix programmed to receive a test signal from the state machine, to apply the test signal to a cable coupling a sensor to the physiological monitor, and to receive a response; and
a programmable load programmed to receive the test signal and the response and to transmit a corresponding voltage;
a comparator on the programmable digital logic block, receiving the voltage from the programmable load, to convert the voltage to a test result, and to transmit the test result to the state machine; and
a communication interface on the programmable digital logic block to transmit the test result from the programmable digital logic block to at least one of a monitor processor and a monitor memory.

2. The monitor of claim 1,
further comprising a local memory block, and
wherein the programmable analog logic block, the programmable digital logic block, and the local memory block occupy a footprint of between 2 and 20 square millimeters ("mm²").

3. The monitor of claim 1 or 2, wherein the communication interface exchanges data with the monitor processor or the monitor memory through a two-wire serial communication bus.

4. A method, comprising:
coupling a cable to a sensor and a physiological monitor;
positioning the sensor to measure a physiological parameter of a patient; and
measuring the physiological parameter while:
activating a first circuit in a programmable switch matrix;
performing a first cable test using the first circuit;
receiving a first test result from the cable;
generating a first fault flag corresponding to the first test result;
transmitting the first test result to at least one of a fault register, a state machine, or a monitor processor.

5. The method of claim 4, wherein at least part of the sensor's measurements of the physiological parameter continue uninterrupted during at least part of the first cable test.

6. The method of claim 4 or 5, wherein the first circuit is activated by being created or modified in a macro model.

7. The method of claim 6, further comprising updating or modifying the macro model *in situ* at an end-user facility.

8. The method of claim 7, wherein:
(i) at least one of the fault register and the state machine is part of a programmable digital logic block, and further comprising updating or modifying the programmable digital logic block *in situ* at an end-user facility; and/or
(ii) further comprising operating of the physiological monitor, wherein the operating begins with a start-up sequence and ends with a shut-down sequence, and further comprising:
loading the macro model from a non-volatile memory during the start-up sequence, and
saving the macro model to a non-volatile memory during the shut-down sequence.

9. The method of any one of claims 4 to 8, further comprising receiving instructions to perform a second cable test, optionally wherein the second cable test differs from the first cable test and includes activation of a second circuit, different from the first circuit, on the programmable analog logic block.

10. The method of claim 9, further comprising receiving instructions to perform a second cable test, wherein the first cable test and the second cable test are part of a plurality of cable tests, each cable test having a corresponding circuit to activate on the programmable analog logic block optionally wherein the plurality of cable tests comprises at least two of a short-circuit test, an open-circuit test, and a ground-fault test..

11. The method of claim 10, further comprising repeating each of the first cable test and the second cable test after one of:
passage of a predetermined time,
completion of a predetermined sequence of other tests or other processes by the monitor; or
selection of the cable test by the monitor processor according to an algorithm or adaptive learning model.

12. The method of claim 10 or 11, wherein the sensor comprises a pair of back-to-back diodes and the plurality of cable tests comprises detecting a short circuit or an open circuit by measuring a direction of current in the cable to or from the back-to-back diodes.

13. A non-transitory computer-readable storage medium containing instructions that, when executed, cause a physiological monitor processor to:
detect whether a sensor is coupled by a cable to a physiological monitor that includes the physiological monitor processor;
decide to test the cable for faults if predetermined conditions are present;
activate a cable fault detector;
select a cable test from a group of tests stored in a memory;
command the cable fault detector to perform the cable test;
receive a pass-or-fail result of the cable test from the cable fault detector; and
add the pass-or-fail result, an identifier of the cable test, and the date and time to a record of cable tests; and
if the pass-or-fail result is a fail, trigger an alert to a user.

14. The non-transitory computer-readable storage medium of claim 13, wherein selecting the cable test is based on the relative frequency of failure types stored in the record of cable tests.

15. The non-transitory computer-readable storage medium of claim 13 or 14, further comprising:
receiving an update to the instructions for operating the cable fault detector;
authenticating the update; and
modifying the instructions according to the update, wherein the modifying includes reprogramming a portion of a programmable analog logic block or a programmable digital logic block of the cable fault detector.
